# NEW EUROPEAN PATENT SPECIFICATION

(11) **EP 2 694 007 B2**
(45) Date of publication and mention of the opposition decision: **27.02.2019**
(45) Mention of the grant of the patent: 05.08.2015
(21) Application number: 13714499.4
(22) Date of filing: 26.03.2013
(51) Int. Cl.: A61F 13/49, A61F 13/15, B26D 1/40

(54) **ELASTIC MEMBER CUTTING ROLL SYSTEM, METHOD, AND ABSORBENT ARTICLE MADE THEREFROM**
SCHNEIDEROLLENSYSTEM FÜR EIN ELASTISCHES ELEMENT UND DARAUS HERGESTELLTER SAUGFÄHIGER ARTIKEL
SYSTÈME DE ROULEAUX DE COUPE D'UN ÉLÉMENT ÉLASTIQUE, PROCÉDÉ, ET OBJET ABSORBANT RÉALISÉ À PARTIR DE CELUI-CI

(30) Priority: 30.03.2012 US 201261617713 P
(43) Date of publication of application: 12.02.2014
(73) Proprietor: The Procter & Gamble Company, Cincinnati, OH 45202 (US)
(72) Inventor: FUJIKAWA, Hiroshi, Akashi Hyogo 674-0093 (JP); OGATA, Takeshi, Akashi Hyogo 674-0093 (JP); UENO, Hiroyuki, Akashi Hyogo 674-0093 (JP); JUGUILON, Norman, Gorospe, Akashi Hyogo 674-0093 (JP)
(74) Representative: Heide, Ute
(86) International application number: PCT/US2013/033775
(87) International publication number: WO 2013/148612

(56) References cited:
- EP-A1- 1 188 427
- EP-A1- 1 354 576
- EP-A1- 2 215 999
- EP-A1- 2 258 329
- EP-A1- 2 260 811
- EP-A2- 1 179 330
- JP-A- 2002 113 042
- JP-A- 2009 172 231
- US-A- 5 683 531
- US-A1- 2003 089 447
- US-A1- 2004 015 146
- US-A1- 2005 230 037
- US-A1- 2007 017 330
- US-A1- 2007 284 048
- US-B2- 7 777 094
- <<<N O N - C I T E D D O C U M E N T>>> Machine-translation of P1
- <<<N O N - C I T E D D O C U M E N T>>> Machine-translation of P3
- <<<N O N - C I T E D D O C U M E N T>>> Published divisional application of P5, EP 1938777 A2
- <<<N O N - C I T E D D O C U M E N T>>> Published parent application of P8, US 2005230037 A1
- <<<N O N - C I T E D D O C U M E N T>>> Application Publication of P10, US 2004133180 A1
- <<<N O N - C I T E D D O C U M E N T>>> Published Divisional Application of P12, EP 1961404 A2

## Description

### FIELD OF THE INVENTION

The present invention relates to apparatuses and methods of cutting certain portions of an elastic member, the elastic member being suitable for use in absorbent articles.

### BACKGROUND OF THE INVENTION

Absorbent articles such as diapers have areas of elasticity to provide a snug fit on the wearer. Such areas of elasticity are provided by elastic members to be used as waist bands and leg bands. In some configurations, it may be advantageous for certain areas of the elastic members to be free of elasticity. For example, it may not be desirous to have elasticity in the front and rear section of the diaper matching the area where an absorbent core overlaps. If elasticity is present in the absorbent core overlapping area, this may be uncomfortable for the wearer, and also cause unnecessary bunching up of material deteriorating the absorbing efficiency of the absorbent core. Furthermore, absorbent articles, especially baby diapers, are commonly designed with graphics on the front and/or rear section of the diaper. Such graphics may provide a connotation to quality of the product, amusement to the wearer and caregiver, and may also be used for educational or training purposes. Absence of elasticity in those sections helps prevent the graphics to be distorted. Meanwhile, it is often desirable to have such elastic member cutting conducted in a cost efficient manner with minimum influence to production speed.

Japanese Patent 4630352B discloses a process of conveying continuous bodies of 2 layers of nonwoven fabric sheets and a continuous body of a plurality of elastic members arranged therebetween, intermittently fixing the continuous elastic members on the nonwoven fabric sheets, and cutting respective elastic members at the non-fixing parts thereof together with the nonwoven fabric sheets. Disclosed are nonwoven fabric sheets cut through at the middle of the non-fixing parts in a perpendicular angle to the elastic member in a linear pattern. Usage of a reinforcement sheet is suggested for supplementing the cut and weakened portion of the nonwoven fabric sheet.

Such usage of a reinforcement sheet adds material and process steps. To eliminate usage of a reinforcement sheet, a number of alternative methods have been suggested.

Japanese Patent 4090158B discloses a waist surrounding elastic member which has the elastic strands finely segmented in the portion where elasticity is not desired. For such fine segmenting, the elastic strands are adhered by hot melt glue to the sheets sandwiching them, and the elastic strands are pressure cut or heat cut before the hot melt glue is completely cooled.

Such process requires providing the area undesirable of elasticity to have much glue, which leads to stiffness of the area. Further, pressure cutting or heat cutting adds complexity to the process. If the elastic strands were to be cut by cutting through the supporting sheet, this would result in the blades picking up glue, which would require additional cleaning processes. On the other hand, elimination of glue increases the risk of allowing the finely cut elastics to escape from the apertures made by the cutting, and lead to safety hazards to wearers.

JP 2008-229007A discloses an elastic cutting system by pressure cutting wherein the blades are not aligned in a line parallel to the axis of the roller, and each blade is slanted. The blades are closely positioned so that even if one blade misses cutting the elastic, another one is available for back up. A random cutting pattern is also taught.

Such process requires many blades meticulously aligned, and when combined with pressure cutting, requires much maintenance of the blades. On the other hand, if the material sandwiching the elastics were cut, such would lead to the original problem of creating a weakened position of the material.

US 2005/023037A discloses a staggered cutting knife system for severing the elastic material. The pair of nonwoven layers sandwiching the elastic material are adhered to each other except in a non-adhering portion, and the elastic material is cut at the non-adhering portion which eventually becomes a garment blank. The knife assemblies are held by respective clamp bars, and designed to burst cut the elastics at a right angle. The clamp bars are further held by a back up wedge, and bolts are used to secure such position of the knife assemblies.

Such knife assembly requires adjusting the interference range by arranging the bolts, and allows for only a narrow process window.

Based on the foregoing, there remains a need for providing a reliable elastic member cutting system which is cost efficient, which has little affect on production speed, and provides a resulting product which is aesthetically pleasing and soft without deteriorating absorbency and usage performance.

### SUMMARY OF THE INVENTION

In one aspect, the present invention relates to an elastic member cutting roll system for cutting a plurality of elastic strands in a region in accordance with claim 1.

In another aspect, the present invention relates to a process of continuously manufacturing an elastic member having an elastic region and a non-elastic region in accordance with claim 4.

The process of the present invention may suitably utilize the elastic member cutting roll system mentioned above.

In yet another aspect, the present invention relates to an absorbent article having a waist opening and two leg openings and continuous in a longitudinal direction and a transverse direction, the article comprising an absorbent main body and a ring-like elastic belt, wherein:
the absorbent main body comprises a liquid pervious topsheet, a liquid impervious backsheet, and an absorbent core disposed therebetween, the absorbent main body has left and right longitudinally continuous side edges, front and back transversely continuous end edges, longitudinally opposing front and back waist panels, and a crotch panel between the waist panels;
the ring-like elastic belt comprises a front belt portion and a back belt portion each comprising an inner sheet, an outer sheet, and a plurality of elastic strands sandwiched between the inner and outer sheets, the elastic strands extending in the transverse direction to provide a continuous elastic ring when the front belt portion and the back belt portion are joined, each front belt portion and back belt portion having transversely continuous proximal and
distal edges, the proximal edge being located closer than the distal edge relative to the crotch panel of the absorbent main body, longitudinally continuous left and right side edges, a central panel, and left and right side panels contiguous with its central panel, each side panel having a longitudinal length defined by the respective side edge of the respective belt portion;
the central panel of the front belt portion is joined to the front waist panel of the absorbent main body, the central panel of the back belt portion is joined to the back waist panel of the absorbent main body, and the respective left and right side panels of the front belt portion and the back belt portion are joined together at or adjacent to the respective left and right side edges to form the waist opening and the two leg openings;
wherein each of the front belt portion and the back belt portion comprises a non-elastic region where the ring-like elastic belt more or less overlaps with the absorbent core, an elastic region defined elsewhere, the non-elastic region having the inner sheet and the outer sheet intermittently adhered with each other without adhering the elastic strands, the elastic region having the elastic strands intermittently adhered between the inner sheet and the outer sheet, the elastic strands in the non-elastic region being deactivated by cutting the elastic strands.

The ring-like waist belt of the article of the present invention may suitably be manufactured by the process mentioned above utilizing the elastic member cutting roll system mentioned above.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:
Figure 1 is a perspective view of one embodiment of an absorbent article of the present invention.
Figure 2 is a top plan view of one embodiment of an absorbent article of the present invention in a flat uncontracted condition showing the inner, body facing surface.
Figure 3 is a cross-sectional view of Figure 2 taken along the line **III-III.**
Figure 4 is a cross-sectional view of Figure 2 taken along the line IV-IV.
Figure 5 is a schematic top plan view of one embodiment of an elastic member of the present invention.
Figure 6 is a schematic view of the process for manufacturing the absorbent article of Figure 1.
Figure 7 is a schematic view of an elastic member cutting process and elastic member cutting roll system of the present invention.
Figure 8 (a), (b) and (c) are a cutting roll system and the resulting knife trail patterns of one embodiment of the present invention.
Figure 9 (a), (b) and (c) are a cutting roll system and the resulting knife trail patterns of the prior art.

### DETAILED DESCRIPTION OF THE INVENTION

Various non-limiting embodiments of the present invention will now be described to provide an overall understanding of the principles of the structure, function, manufacture, and use of the apparatuses, methods, and articles disclosed herein. One or more examples of these non-limiting embodiments are illustrated in the accompanying drawings. Those of ordinary skill in the art will understand that the apparatauses and methods specifically described herein and illustrated in the accompanying drawings are non-limiting example embodiments and that the scope of the various non-limiting embodiments of the present invention are defined solely by the claims. The features illustrated or described in connection with one non-limiting embodiment may be combined with the features of other non-limiting embodiments. Such modifications and variations are intended to be included within the scope of the present invention.

### Cutting Roll System

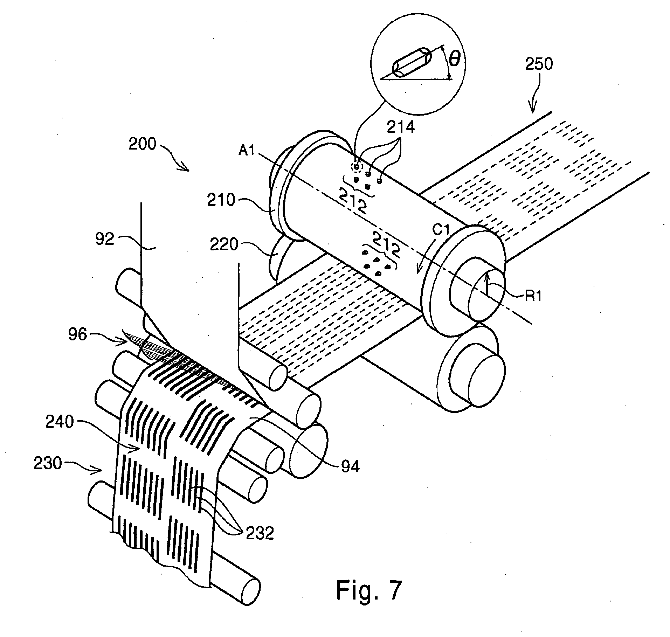

The present invention relates to a cutting roll system for cutting a certain region of an elastic member having a plurality of elastic strands. Referring to Figure 7, the cutting roll system 200 comprises a cutting roll 210 adapted to rotate about a roll axis, and an anvil roll 220. As discussed in further detail below, the cutting roll 210 comprises a knife block 212 having a plurality of knife edges 214. The anvil roll 220 is adjacent the cutting roll and engages the knife edges 214 when in cutting action. The elastic strands 96 of the elastic member 250 to be cut is advanced to the cutting roll system 200 in the roll circumferential direction C1. The elastic strands 96 may be attached to a continuous substrate on either or both sides facing the cutting roll 210 or the anvil roll 220. The continuous substrate facing the cutting roll 210, when present, may be chosen such that the knife edge 214 may cut through both the substrate and elastic strand in one cutting action. The continuous substrate may be a sheet.

The knife edges 214 are secured to the roll in groups, each group for cutting a certain area or region, referred to as a knife block herein. A knife block 212 may be made of a particular part for engaging with the cutting roll 210, or provided in multiple parts or provided as individual knife edges 214 embedded in the cutting roll. In some configurations, the knife block 212 is provided as a replaceable part.

As used herein, the term "row" refers to a series of components such as knife edges aligned in the roll axis direction A1, and the term "column" refers to a series of components aligned in the roll circumferential direction C1.

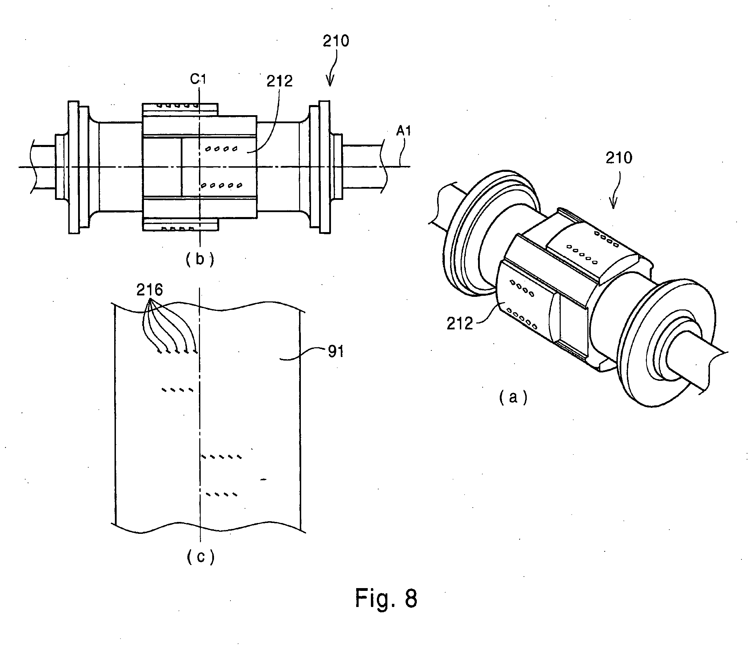

Within the knife block 212, the knife edges 214 are provided in at least two rows and each row having at least two knife edges arrayed linearly parallel to the axis roll, and each row of knife edges circumferentially spaced apart from each other. The configuration of the knife edges 214 in one knife block 212 is positioned such that each elastic strand 96 in a given region is cut only once. Specifically, the at least two rows of knife edges 214 are staggered with each other in the roll axis direction A1, and each knife edge 214 is positioned such that the same knife edge 214 does not cut two or more elastic strands 96 at the same time. The embodiment of Figure 7 shows two or three knife edges 214 arrayed in two rows. However, it is to be appreciated that the number of knife edges and/or rows can be varied depending on the desired cutting configuration. In the embodiment of Figure 8(a) and 8(b) showing the same cutting roll 210 from different angles, the two rows of four or five knife edges are spaced apart in the roll circumferential direction C1 of the knife block 212 so that the knife trail pattern 216 results as shown in Figure 8(c).

Referring to Figure 7, the knife edges 214 of the present cutting roll system 200 are for cutting the elastic strands 96 and any substrate material between the knife edge 214 and elastic strand 96. The knife edges may be a rotary die cutter. The knife edge 214 useful herein has a relatively small land area for contacting the cutting surface, typically no more than 100 µm. Each knife edge 214 is oriented at an angle which does not match with the the roll axis direction A1 or the roll circumferential direction C1. The knife edges 214 may be oriented at an angle (θ) of from about 10 to about 45 degrees, or from about 15 to about 35 degrees to the roll axis direction A1. Further, each knife edge 214 projects outward from the roll center in alignment with the roll radial direction R1. By providing the knife edges in such an angle, the pressure at the contact point of the knife edge may be distributed in the roll circumferential direction C1, such that the cutting accuracy and pressure fluctuation control may be improved. Consistent cutting accuracy and control of pressure varation may be important elements for preventing undesirable vibration at the contact point. In addition, minimization of such undesirable vibration may be important for maintaining standard quality cutting at high speed. Further, in that pressure on the knife edge 214 is distributed, there may be less damage to the knife edge 214, thus knife life cycle may be prolonged. Still further, in that the knife edges 214 are oriented at an angle away from the roll circumferential direction C1, the cutting roll system 200 may cut elastic strands 96 having smaller intervals between each other. The knife edges 214 may be made by tungsten carbide, tool steel, or any other suitable material.

The knife edges 214 within a knife block may all be oriented in the same angle as in Figure 7, or oriented in different angles. Having the knife edges 214 oriented in the same angle, or in a symmetric angle with respect to the roll circumferential direction C1, may be advantageous for controlling the pressure fluctuation upon cutting.

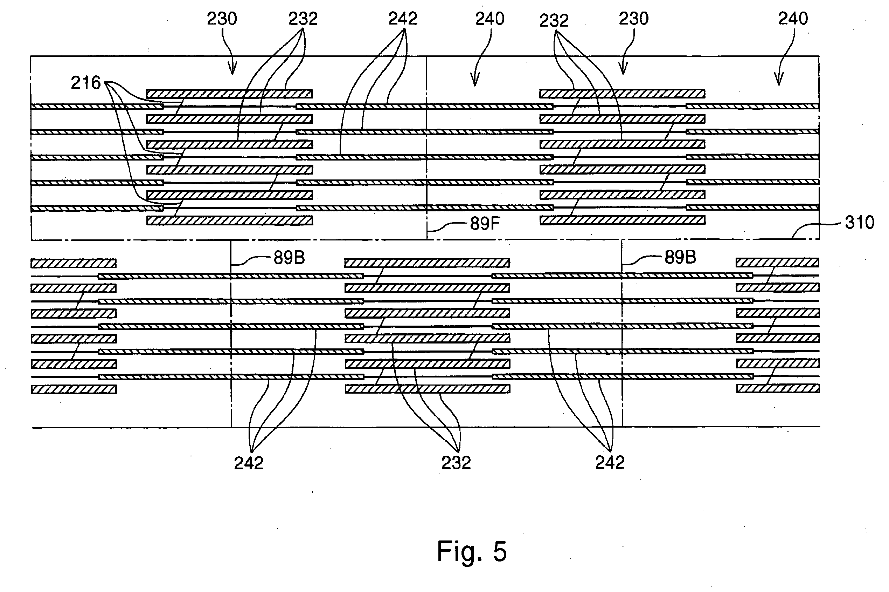

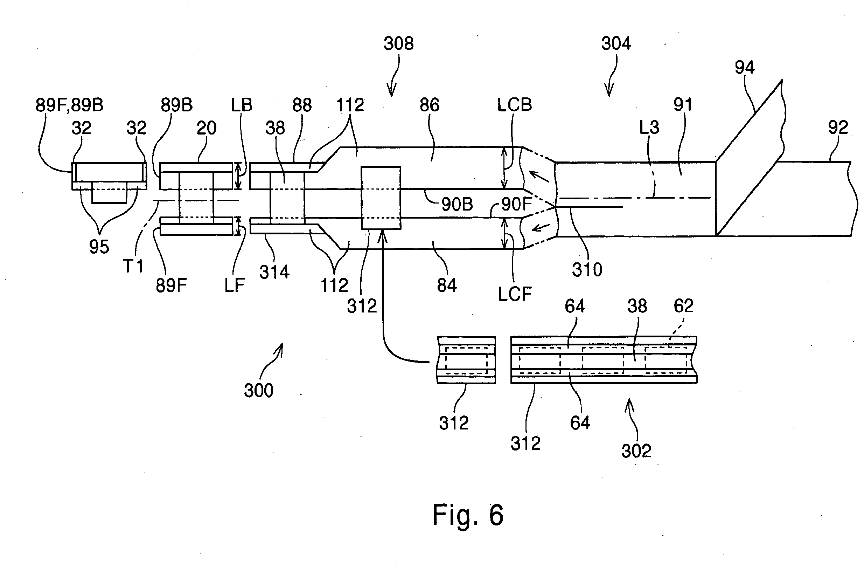

The cutting roll system 200 of the present invention has a plurality of knife blocks 212. Providing a plurality of knife blocks 212 is advantageous for creating multiple non-elastic regions per roll. With the plurality of knife blocks 212 provided in one roll, each knife block 212 has knife edges 214 oriented at an angle which does not match with the roll axis direction A1 or the roll circumferential direction C1. Further, the knife blocks 212 are staggered with each other in both the roll axis direction A1 and the roll circumferentical direction C1 such that no more than one knife block 212 is contacting the anvil roll at the same time. In the embodiment of Figure 7, four knife blocks 212 are positioned in two columns, wherein each knife block 212 is spaced apart 90 degrees in the roll circumferentical direction C1, wherein there are no two knife blocks 212 aligned in the roll axis direction A1. Such positioning of the knife blocks 212 is advantageous for controlling the pressure fluctuation upon cutting. Further, by providing two or more columns of knife blocks 212 on the same cutting roll, two or more columns of regions of the elastic members 96 may be cut, resulting in non-elastic regions 230 spaced apart in the cross machine direction as well as the machine direction, as shown in Figure 5. Such is advantageous in that concentration of the cut and weakened regions can be avoided, thus it is easier to handle the obtained continuous elastic member. What is meant by handling is, for example, cutting the continuous elastic member apart in the machine direction to provide two continuous sections along cut line 310 as shown in Figure 6. To the contrary, if the cut and weakened regions were aligned in the cross machine direction, there may be higher risk of undesirable elongation, sag, tracking, neck down, or even failure in the concentrated weakened region.

### Manufacturing Method

The present invention also relates to a process for continuously manufacturing an elastic member having an elastic region and a non-elastic region, the elastic member made of two layers of continuous sheets and a plurality of elastic strands sandwiched between the two layers of continous sheets. Referring to Figure 7, the process relates to advancing two layers of continuous sheets 92, 94 and a plurality of elastic strands 96 in the machine direction, stretching the elastic strands 96 in the machine direction, wherein certain portions of the three components are adhered with one another to make a continuous elastic member 250. The continuous sheets 92, 94 and the elastic strands 96 are advanced in the machine direction, the direction matching the roll circumferential direction C1 of the cutting roll 210 at which the elastic strands 96 are eventually cut. The adhesion may be provided by holt-melt adhesive, heat, ultrasound, or any other method known in the art.

Referring to Figure 5, the portions for which adhesion is provided and the position of cutting 216 thereafter define the elastic region 240 and non-elastic region 230 of the continuous elastic member. The elastic strands may be positioned in any interval between each other, and any variation of stress strain per strand, or variation of stress strain on the same strand within the elastic region, according to the need of the resulting elastic member.

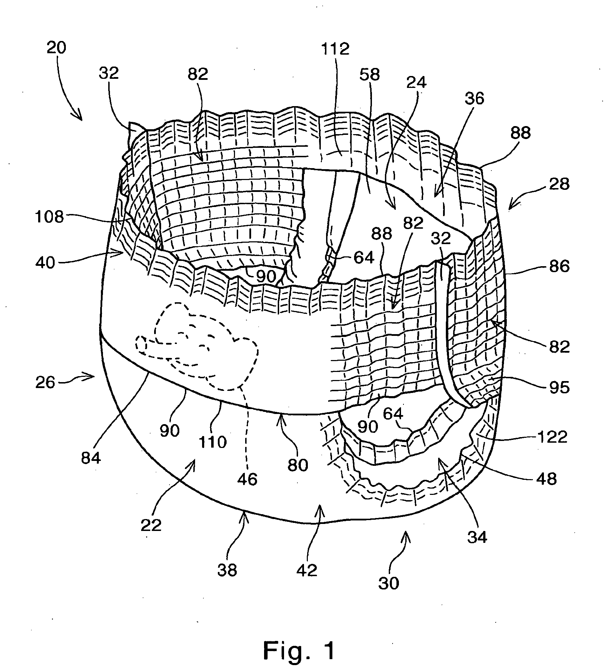

The elastic region 240 is created by intermittently adhering 242 the elastic strands between the two layers of continuous sheets. The elastic strands in the elastic region 240 are not cut. By such steps and after the tension stretching the elastic strands are eventually removed, the elastic strands return to their relaxed state to create gathers with the two layers of continuous sheets, such as shown in 82 of Figure 1.

The non-elastic region 230 is created by intermittently adhering 232 the two layers of continuous sheets with each other without adhering the elastic strands, and cutting the elastic strands 216 within such region. By such steps, the elastic strands which remain unadhered are deprived of their tension when cut, and the region is left with two layers of continuous sheets in more or less their original size/tension adhered to each other in limited areas. The non-elastic region 230 created by the present process has adhesion only in limited areas, thus more or less maintains softness of the original sheet materials. A portion of the elastic region, such as areas adjacent of the non-elastic region, may have the two layers of continuous sheets adhered with each other.

The elastic regions and non-elastic regions are each intermittently spaced along the machine direction, the non-elastic regions being separated by the elastic regions from each other.

Referring to Figures 5 and 7, the adhered components having had gone through the aforementioned steps are then advanced through a cutting roll system 200 for cutting the elastic strands 96 only in the non-elastic region 230. The cutting roll system 200 mentioned above may be suitably used for the purpose of this step. Each elastic strand 96 per non-elastic region is cut no more than once in a staggered manner 216. By such cutting, the strength of the continuous sheets 92, 94 of the non-elastic region 230 is less affected. The continuous sheet facing the knife edge may be cut through for cutting the elastic strands. The non-elastic region 230 may be cut towards the two edges of the region in the machine direction, so that the knife trail patterns 216 are disposed away from the center of the non-elastic region 230. The knife block of Figures 8(a) and 8(b) may be used for this purpose. With such configuration, the strength of the non-elastic region 230 is more or less maintained that there is no additional sheet required for reinforcing the non-elastic region 230. Other knife edge arrangements are also useful for providing different knife trail patterns 216. Referring to Figure 1, the knife trail patterns 216 may be coordinated with the graphics 46, particularly when the knife trail patterns 216 and graphics 46 are observable together. For example, the knife trail patterns 216 may avoid the graphics 46, or the knife trail patters 216 may coincide with a certain element of the graphic 46. The graphic may be printed directly on either or both the continuous sheets, or other material observable through the continuous sheets.

The present process is particularly useful for making elastic members to be used as belts, waist bands, leg bands, head bands, and wrist bands of wearable articles having an elastic region and a non-elastic region. The continuous sheets may be the fabrics or non-woven sheets for making the wearable article, wherein the non-elastic region matches with the area wherein elasticity is not desired. By using a cutting roll system having a plurality of knife blocks in the roll axis direction, more than one continuous elastic members using the same continuous sheets may be made by the present process side by side as in Figure 5. The plurality of continuous elastic members may be the same elastic members made in plurality, or different types of elastic members simultaneously. Such different elastic members may be different in the length of the elastic member, in the length of the continuous sheet in the cross-machine direction, number of elastic strands, the interval of elastic strands, stress strain on the elastic strands, type of elastic strands, length of the non-elastic region in the machine direction, length of the non-elastic region in the cross-machine direction, length of the elastic region in the machine direction, length of the elastic region in the cross-machine direction, or any other configuration. The present process is particularly useful for making elastic members of an absorbent article in a cost effective manner.

### Absorbent Article

As used herein, the term "absorbent article" refers to articles of wear to absorb and contain various exudates such as urine, feces, and menses discharged from the body, and may be in the form of pants, taped diapers, incontinent briefs, feminine hygiene garments, and the like. The absorbent article made partly by the elastic member cutting roll system or the continuous process described above may have a defined waist opening and a pair of leg openings and which are pulled onto the body of the wearer by inserting the legs into the leg openings and pulling the article up over the waist. An example absorbent article 20 is shown in Figure 1.

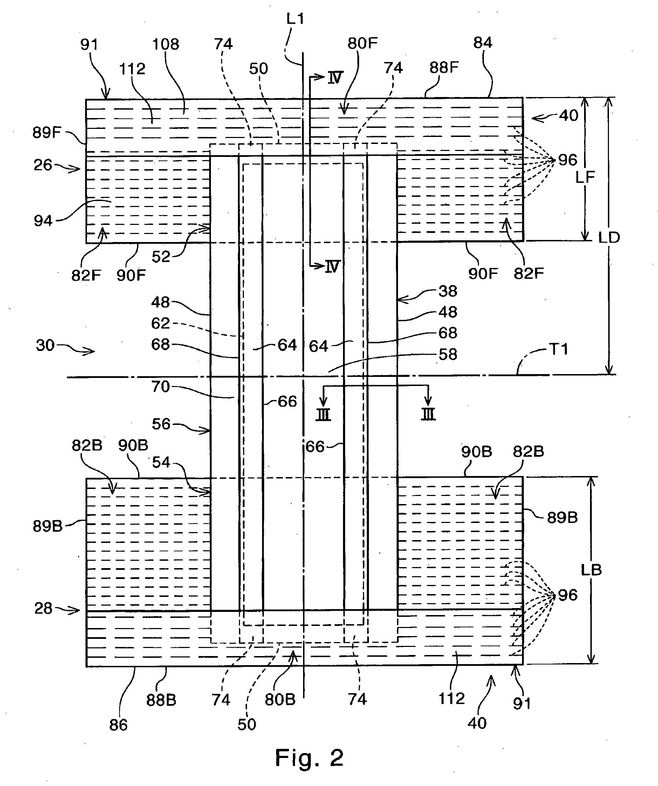

Figure 1 is a perspective view of the absorbent article 20 of the present invention and Figure 2 is a top plan view of the same article in its flat uncontracted condition showing the inner, body-facing surface. The absorbent article 20 has a longitudinal centerline L1 and a transverse centerline T1. The absorbent article 20 has an outer surface 22, an inner surface 24 opposed to the outer surface 22, a front region 26, a back region 28, a crotch region 30, and seams 32 which join the front region 26 and the back region 28 to form two leg openings 34 and a waist opening 36. The absorbent article 20 comprises an absorbent main body 38 (hereinafter may be referred to as "main body") to cover the crotch region of the wearer, a ring-like elastic belt 40 (hereinafter may be referred to as "elastic belt" or "belt") extending transversely about the waist opening 36, and an outer cover layer 42 to cover the main body 38. The elastic belt 40 defines the waist opening 36. The elastic belt 40 and the main body 38 and/or the outer cover layer 42 jointly define the leg opening 34. Alternatively, the elastic belt 40 and the outer cover layer 42 may jointly define the leg opening 34.

The absorbent main body 38 absorbs and contains body exudates disposed on the main body 38. In the embodiment shown in Figure 2, the main body 38 has a generally rectangular shape, left and right longitudinally extending side edges 48 (hereinafter may be referred to as "longitudinal side edge") and front and back transversely extending end edges 50 (hereinafter may be referred to as "transverse end edge"). The main body 38 also has a front waist panel 52 positioned in the front region 26 of the absorbent article 20, a back waist panel 54 positioned in the back region 28, and a crotch panel 56 between the front and back waist panels 52, 54 in the crotch region 30.

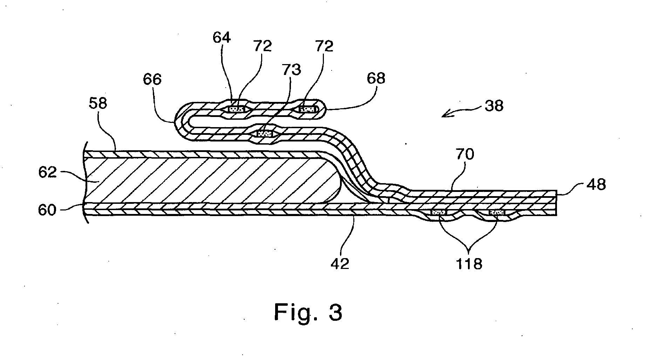

The main body 38 comprises a liquid pervious topsheet 58, a liquid impervious backsheet 60 and an absorbent core 62 disposed therebetween. The main body 38 may additionally comprise a barrier leg cuff 64 disposed along the longitudinal side edge 48. The barrier leg cuff 64 provides improved containment of liquids and other body exudates in the crotch region 30. The barrier leg cuff 64 shown in Figure 3 comprises a single layer of material which is folded into two layers. The barrier leg cuff 64 extends from the longitudinal side edge 48 toward the longitudinal centerline L1 and then is folded along the folding line 66 back toward the longitudinal side edge 48. The barrier leg cuff 64 has two barrier cuff elastic materials 72 adjacent the distal portion 68 and one barrier cuff elastic material 73 adjacent the proximal portion 70 of the barrier leg cuff 64. The proximal portion 70 of the barrier leg cuff 64 is joined to the backsheet 60 adjacent the longitudinal side edge 48. The portion of the barrier leg cuff 64 along the folding line 66 and the distal portion 68 are free from attachment to any portion of the main body 38 in the crotch panel 56 such that the barrier leg cuff 64 stands up toward the wearer's body. The transverse end 74 of the barrier leg cuff 64 is joined to the topsheet 58 adjacent the folding line 66 by an attachment means 76 which may be any known means such as an adhesive and is joined onto the barrier leg cuff 64 itself along the distal portion 68 by an attachment means 78 which may be any known means such as an adhesive. Many other configurations for the barrier leg cuffs are possible, including those having less barrier cuff elastic materials and/or folding lines.

The liquid pervious topsheet 58 may be positioned adjacent the body-facing surface of the absorbent core 62 and may be joined thereto and/or to the backsheet 60 by any attachment means known in the art. The liquid impervious backsheet 60 is generally that portion of the absorbent article 20 positioned adjacent the garment-facing surface of the absorbent core 62 and prevents the exudates absorbed and contained therein from soiling articles that may contact the absorbent article 20. The absorbent core is positioned between the topsheet 58 and the backsheet 60 and absorbs and retains liquids such as urine and other certain body exudates. The topsheet 58, the backsheet 60 and the absorbent core may be manufactured by any known materials. Suitable topsheet materials may include porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. Suitable backsheet materials may include breathable materials that permit vapors to escape from the absorbent article while still preventing exudates from passing through the backsheet. Suitable absorbent core materials may include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

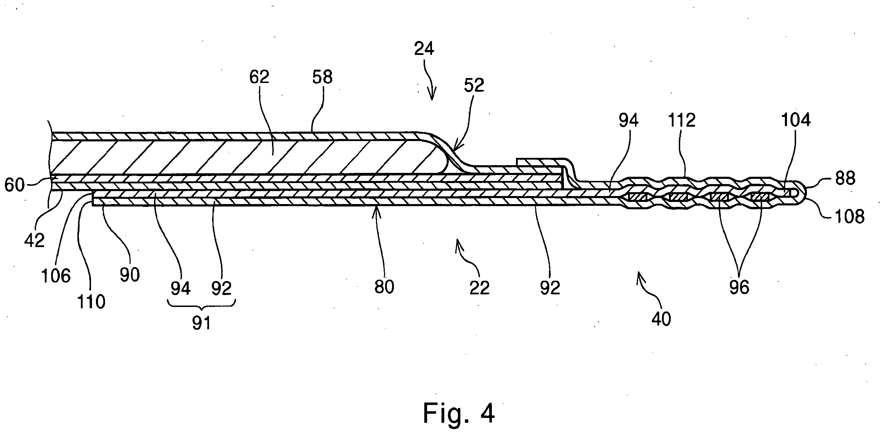

The outer cover layer 42 as well as the inner and outer sheets 92, 94 of the elastic belt may suitably comprise a single layer of nonwoven web of synthetic fiber, which may be hydrophobic, non-stretchable nonwoven material. However, the outer cover layer 42 is not continuous with the inner sheet 94 or outer sheet 92 of the elastic belt. Figure 4 shows a cross section view of a combination of a ring-like elastic belt and absorbent main body of the present invention. The elastic belt 40 comprises an inner sheet 94, an outer sheet 92, and a plurality of elastic strands 96 sandwiched between the inner and outer sheets. Neither the inner sheet 94 or outer sheet 92 extends into the crotch region 30 of the absorbent article, as also shown in Figure 1. Instead, the outer cover layer 42 comprising a single layer of nonwoven is disposed in the crotch region 30. This structure is less costly and allows the crotch region 30 of the absorbent article to be less bulky. The outer cover layer 42 comprising a nonwoven material also provides an integral cloth-like appearance together with the ring-like elastic belt 40 comprising a nonwoven material.

Referring to Figures 1 and 2, the ring-like elastic belt 40 extends transversely about the waist opening 36 of the absorbent article 20 and acts to dynamically create fitment forces and to distribute the forces dynamically generated during wear. The elastic belt 40 comprises a front belt portion 84 and a back belt portion 86 (hereinafter may be referred to as "front and back belt portion 84, 86). Each of the front belt portion 84 and the back belt portion 86 has a central panel 80F, 80B and side panels 82F, 82B contiguous with the central panel 80F, 80B and positioned transversely outward from the central panel 80F, 80B. Herein, a portion of a front member and a portion of a back member may be indicated by a reference number with "F" suffix and "B" suffix, respectively, as necessary. Therefore, the "central panel 80F, 80B" for example indicates the "front central panel 80F" and the "back central panel 80B". The "central panel 80" also may mean the "front central panel 80F" and the "back central panel 80B". Each of the front belt portion 84 and the back belt portion 86 has a transversely extending distal edge 88F, 88B, a transversely extending proximal edge 90F, 90B, and longitudinally extending left and right side edges 89F, 89B. Herein, the term "proximal" is used to indicate the position of a "proximal" portion being closer relative to the crotch panel of the main body than the position of a "distal" portion. Therefore, the proximal edge 90F, 90B is located closer than the distal edge 88F, 88B relative to the crotch panel 56 of the main body 38. The front and back belt portions 84, 86 are joined at or adjacent the side edges 89F, 89B at the seams 32 to form a absorbent article having a waist opening 36 and two leg openings 34. The front central panel 80F may partly or entirely overlap with the front waist panel 52 of the main body 38. The back central panel 80B may partly or entirely overlap with the back waist panel 54 of the main body 38. However, the central panel 80F, 80B does not extend into the crotch panel 56 of the main body 38 and is not disposed in the crotch panel 56. In the embodiment shown in Figure 2, the central panel 80F, 80B partly overlaps with and is joined to the front waist panel 52 and the back waist panel 54, respectively.

The ring-like elastic belt 40 comprises a front belt portion 84 and a back belt portion 86 each comprising an inner sheet, an outer sheet, (hereinafter also collectively "belt sheets") and a plurality of elastic strands 96 sandwiched between the inner and outer sheets, the elastic strands 96 extending in the transverse direction to provide a continuous elastic ring when the front belt portion and the back belt portion are joined. The front belt portion 84 and the back belt portion 86 may comprise the same or different materials and/or may have the same or different structures. For cost efficiency, the front and back belt portions 84, 86 may be made of the same materials and continuously at the same time according to the continous process of the present invention. In the embodiment of Figure 2, the front belt portion 84 and the back belt portion 86 are made of the same inner and outer sheets. Referring to Figure 4, the inner sheet 94 has a transversely extending distal end 104 and a transversely extending proximal end 106. The outer sheet 92 has a transversely extending distal end edge 108 and a transversely extending proximal end edge 110. The inner sheet 94 is almost coextensive with the contour of the front and back belt portion 84, 86. Alternatively, the inner sheet 94 may be smaller than the size of the front and back belt portion 84, 86. The outer sheet 92 of the belt layer 91 may be longer than the size of the inner sheet 94 in the longitudinal direction and an end flap 112 of the outer sheet 92 is folded to cover the distal end 104 of the inner sheet 94 at the waist opening 36 and to form a distal end edge 108 of the outer sheet 92. The inner sheet 94 of the belt layer 91 may also have an end flap which may be folded together with the end flap 112 of the outer sheet 92. The end flap of the inner sheet 94 may be longer or shorter than or equal to the end flap of the outer sheet 92. Alternatively, the end flap 112 may be eliminated such that the outer sheet 92 terminates at the waist opening 36 to form the distal end edge 108. In the embodiment shown in Figures 2 and 4, the distal end edge 108 and the proximal end edge 110 of the outer sheet 92 correspond to the distal edge 88 and the proximal edge 90 of the front and back belt portion 84, 86, respectively. The outer sheet 92 surrounded by the distal end edge 108 and the proximal end edge 110 defines the shape of the front and back belt portion 84, 86 in the embodiment shown in Figures 2 and 4.

Suitable material for the inner and outer sheets of the front and back belt portion 84, 86 can be manufactured from a wide range of materials such as plastic films; apertured plastic films; stretchable or non-stretchable, woven or nonwoven webs of natural materials (e.g., wood or cotton fibers), synthetic fibers (e.g., polyolefins, polyamides, polyester, polyethylene, or polypropylene fibers), or a combination of natural and/or synthetic fibers; or coated woven or nonwoven webs; or bi-component fibers of polyethylene and/or polypropylene. The belt may comprise a nonwoven web of synthetic fibers.

Referring to Figure 4, the outer cover layer 42 is disposed on the outer surface 22 of the absorbent article 20 and covers the crotch panel 56 of the absorbent main body 38. The outer cover layer 42 may extend into and cover the front waist panel 52 and the back waist panel 54 of the main body 38. The outer cover layer 42 is directly joined to and covers the liquid impervious backsheet 60 of the main body 38. The central panel 80 of the front and back belt 84, 86 portion (explained herein below) is joined to the front waist panel 52 and the back waist panel 54 of the main body 38 through the outer cover layer 42. Thus, the outer cover layer 42 is sandwiched between the front and back belt portion 84, 86 and the liquid impervious backsheet 60 of the main body 38. In the embodiment shown in Figure 4, the outer cover layer 42 is coextensive with the liquid impervious backsheet 60. The leg elastic material 118 is disposed so as to extend generally longitudinally along the longitudinal side edge 48 of the main body 38. The leg elastic material 118 may be disposed at least in the crotch region 30 of the absorbent article 20 or may be disposed along the entirety of the longitudinal side edge 48.

The plurality of elastic strands 96 of the front and back belt portions are disposed at a certain interval in the longitudinal direction. The interval may be constant or different, and the stress strain on the elastic strands may be changed per strand. The front belt portion 84 and the back belt portion 86 each comprise a non-elastic region where the ring-like elastic belt overlaps with the absorbent core, an elastic region defined elsewhere. Absence of elasticity in those belt portions more or less overlapping with the absorbent core helps prevent bunching up of the absorbent core, such that the absorbing efficiency of the absorbent core may be maintained, and the absorbent article is comfortable when worn. Such non-elastic region and elastic region is made by: the non-elastic region having the inner sheet and the outer sheet intermittently adhered with each other without adhering the elastic strands, the elastic region having the elastic strands intermittently adhered between the inner sheet and the outer sheet, the elastic strands in the non-elastic region being deactivated by cutting the elastic strands. The elastic strands in the non-elastic region may be cut by the manufacturing method described hereinabove, wherein each elastic strand per non-elastic region is cut no more than once in a staggered manner. With such configuration, the strength of the non-elastic region is more or less maintained that the non-elastic region of the ring-like elastic belt may be free ot any other sheet. By any other sheet, what is meant is reinforcement sheets, graphic patch sheet, and others, which are disposed between the inner sheet and the outer sheet.

Referring to Figure 2, the front side panel 82F has a longitudinal length LF defined by the front side edge 89F of the front belt portion 84 and the back side panel 82B has a longitudinal length LB defined by the back side edge 89B of the back belt portion 86. In one embodiment, the front belt portion 84 and the back belt portion 86 are formed such that the longitudinal lengths LB of the back side panels 82B of the back belt portion 86 are greater than the longitudinal lengths LF of the front side panels 82F of the front belt portion 84. The front belt portion 84 and the back belt portion 86 are formed by cutting a belt layer web along a cut line. The cutting line may be any shape such as straight line, curve, wavy, or sinusoidal, according to the needs of the front and back belt portions. In some configurations, the cutting line may accommodate the proximal edge 90F and back proximal edge 90B to be in a form of corresponding relationship, for example, the proximal edge 90F having a concave curve conforming to a projected curve of the back proximal edge 90B (not shown). In one embodiment, the cutting line defining 90F and 90B is a straight line parallel to the front distal edge 88F and back distal edge 88B (Figure 5).

In one embodiment, the back belt portion 86 has a greater longitudinal length LB between the back distal edge 88B and the back proximal edge 90B along its entire width of the back belt portion 86 in the transverse direction than the longitudinal length LF of the front belt portion 84 between the front distal edge 88F and the front proximal edge 90F (Figures 1, 2, and 6). In such embodiment, when the absorbent article is assembled to form the waist opening 36 and the leg openings 34, the absorbent article 20 is folded along the transverse centerline T1 such that the front distal edge 88F is aligned with the back distal edge 88B. The front side edge 89F is also aligned with a portion of the back side edge 89B. Then the front belt portion 84 and the back belt portion 86 are joined at or adjacent the front and back side edges 89F, 89B at the seams 32. The front and back proximal edges 90F, 90B, however, are not aligned to one another. The back proximal edge 90B is disposed longitudinally closer than the front proximal edge 90F relative to the transverse center line T1 such that the proximal portion of the back side panel 82B extends toward the crotch panel 56 of the main body 38 beyond the front proximal edge 90F. Thus, the proximal portion of the back side panel 82B provides a buttock cover 95. The side edge of the proximal portion is not joined to anywhere and is free from attachment.

The dimension of the buttock cover 95 should be carefully selected to provide an effective function of buttock cover. Referring to Figure 2, the ratio of the longitudinal length LB of the back side edge 89B to the longitudinal length LF of the front side edge 89F may be between about 1.1 and about 2.0, or between about 1.1 and about 1.5 in a laid out flat configuration of the article. The longitudinal length LC is the difference between LB and LF to provide the buttock cover 95. The ratio of the length LC to the length LF may be between about 0.1 and about 1.0, or between about 0.1 and about 0.5 in a laid out flat configuration of the article. The longitudinal article length LD is the distance from the distal edge 88 to the transeverse center line T1 when the article is laid out flat. The longitudinal length LB of the back side edge 89B may be between about 50 % and 100 %, or between about 60 % and about 80 % of the longitudinal article length LD when the article is laid out flat. The article having these dimension characteristics provides an effective buttock cover without hindering the wearer from inserting legs into the leg opening.

The buttock cover 95 may comprise one or more strands 96 having greater contraction force than the remainder of the belt elastic strands on the back side panel 82B. The greater contraction force at the buttock cover helps gathering the belt layer 91 to provide an aesthetic appearance with the buttock cover 95, as in Figure 1.

The front belt portion 84 and the back belt portion 86 are formed by cutting the belt layer web along a cut line. Figure 6 shows a schematic view to explain the process for forming the absorbent article 20. The process 300 primarily comprises three sections; a main body forming section 302, a belt forming section 304 and an assembly section 308. Since Figure 6 is a schematic view, it should be noted that various parts of the absorbent article have been omitted, such as the belt elastic strands and the leg elastic material.

The main body forming process 302 combines elements forming the main body 38 such as the topsheet 58, the backsheet 60, the absorbent core 62 and the barrier leg cuff 64 such that the absorbent core 62 is sandwiched between the topsheet 58 and the absorbent core 62. The outer cover layer 42 (not shown in Figure 6) is joined to the backsheet 60 (not shown in Figure 6) and the leg elastic material 118 (not shown in Figure 6) is sandwiched between the backsheet 60 and the outer cover layer 42. These elements are joined to each other by any known means such as adhesives or heat bonding to form an intermediate main body 312. The intermediate main body 312 is then cut into the individual intermediate main body 312. The individual intermediate main body 312 is turned by 90 degrees and fed into the assembly section 308.

The belt forming section 304 combines the outer sheet web 92, the inner sheet web 94, and plurality of elastic strands to form a continuous belt layer web 91. The continuous belt layer web may be manufactured according to the process of manufacturing an elastic member having elastic regions and non-elastic regions as explained above and illustrated in Figure 7. The continuous belt layer web 91 is cut along a straight cut line 310 which corresponds to the proximal edge 90F, 90B to form a continuous front belt web 84 and a continuous back belt web 86. The cut line 310 is biased from the longitudinal centerline L3 of the continuous belt layer web 91 to differentiate the length LCF of the continuous front belt web 84 and the length LCB of the continuous back belt web 86 in the cross machine direction. Then the continuous front belt web 84 and the continuous back belt web 86 are separated from one another. When the non-elastic regions of the continuous front belt web 84 and the continuous back belt web 86 are provided in a staggered manner, the longitudinal center line L1 of the front and back belt webs are aligned (not shown), while sending the webs to the next article assembling section.

The article assembling section 308 combines the individual intermediate main body 312 with the continuous front belt web 84 and the continuous back belt web 86. The individual intermediate main body 312 is placed on the continuous front and back belt webs 84, 86 at a predetermined interval to provide the side panel between each of the individual intermediate main bodies 312. The end flap 112 of the front and back belt webs 84, 86 is folded inwardly along the distal edge 88 to form a continuous absorbent article assembly 314 comprising the main body 38, the outer cover layer 42 (not shown in Figure 6) and the front and back belt webs 84, 86. The continuous absorbent article assembly 314 thus formed is cut into each individual absorbent article 20. The individual absorbent article 20 has the longitudinal length LB of the back side edge 89B being greater than the longitudinal length LF of the front side edge 89F. The individual absorbent article 20 is then folded along the transverse centerline T1 in the crotch region and the front and back belt 84, 86 is joined at the seam 32 adjacent the side edges 89F, 89B to form the waist opening and the leg openings. The buttock cover 95 is also formed as shown in Figure 6 without requiring trimming any portion of the belt layer web.

The obtained absorbent article is aesthetically pleasing and soft without deteriorating absorbency and usage performance, and can be made in a cost efficient manner at high speed.

### Example 1

An absorbent article according to the present invention was made using the cutting roll system with cutting roll 210 and knife blocks 212 as shown in Figures 8 (a) and (b), such that the knife trail patterns 216 in the non-elastic regions of the front belt portion and back belt portion resulted as shown in Figure 8(c). The adhesions in the front belt portion and back belt portion required for making the elastic regions and non-elastic regions were provided by hot-melt adhesive glue.

### Comparative Example

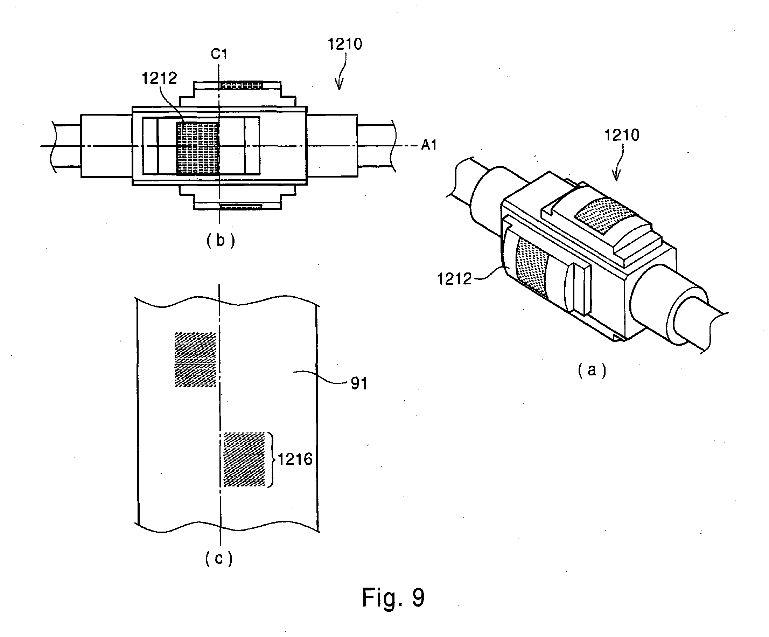

An absorbent article with the same materials and dimensions as Example 1 was made except using the cutting roll system with cutting roll 1210 and knife blocks 1212 as shown in Figures 9 (a) and (b), such that the knife trail patterns 1216 in the non-elastic regions of the front belt portion and back belt portion resulted as shown in Figure 9(c), and further having the entirety of the non-elastic regions adhered with hot-melt adhesive glue. The dimensions of knife edges of the knife blocks 1212 and its resulting knife trail pattern of Figures 9(a), (b), and (c) are not accurately shown, as being very fine.

### Appearance and Sensory Evaluation

Fifteen (15) panelists who regularly diaper their children are given both Example 1 sample and Comparative Example sample, each having graphics in the non-elastic regions of the front and back waist panels, and asked to compare the appearance and feel of the samples. Panelists were asked the questions in Table 1 and to rate each sample in 5 scales: 5 totally agree, 4 somewhat agree, 3 neutral, 2 somewhat disagree, and 1 disagree. The average score is summarized in Table 1.

**Table 1**

| Question | Example 1 a | Comparative Example b |
|---|---|---|
| Diaper has good quality | 3.8 | 4.0 |
| Is a well designed diaper | 3.8 | 3.7 |
| Graphics look good | 3.8 | 3.5 |
| Better softness around graphics on the front | 4.4a* | 3.3 |
| Better softness around graphics on the back | 4.3a* | 3.3 |
| There is leakage concern for the diaper | 1.5 | 1.5 |
| *Statistically significant over Comparative Example with 95% confidence level. The remainder questions do not indicate statistical significance in rating. | | |

The Example 1 article of the present invention has significantly better softness in the non-elastic regions of both the front and back waist panels, while being as aesthetically pleasing and connoting similar quality compared to the Comparative Example article.

## Claims

1. A cutting roll system (200) for cutting a plurality of elastic strands (96, 216) in a region, the cutting roll system (200) comprising:
an anvil roll (220); and
a cutting roll (210) adjacent the anvil roll (220), the cutting roll (210) adapted to rotate about a roll axis, and wherein the cutting roll (210) comprises a knife block (212);
wherein the knife block (212) comprises at least two rows of knife edges (214), each row having at least two knife edges arrayed linearly parallel to the roll axis; each row of knife edges (214) circumferentially spaced apart from each other;
each knife edge (214) oriented at an angle which does not match with the roll axis direction (A1) or a roll circumferential direction (C1), the roll circumferential direction (C1) being perpendicular to the roll axis direction (A1);
the knife edges (214) in the same knife block (212) staggered with each other such that each elastic strand (96, 216) in the region is cut no more than once,
wherein the cutting roll system (200) comprises a plurality of knife blocks (212) on the cutting roll (210), the knife blocks (212) staggered with each other in both the roll axis direction (A1) and roll circumferential direction (C1) such that no more than one knife block (212) is contacting the anvil roll (220) at the same time.

2. The cutting roll system (200) of Claim 1 wherein the knife edge (214) is a rotary die cutter.

3. The cutting roll system (200) of Claim 1 wherein the knife edges (214) in the same knife block (212) are oriented in the same angle to the roll axis.

4. A process of continuously manufacturing an elastic member (250) having an elastic region (240) and a non-elastic region (230) comprising the steps of:
advancing two layers of continuous sheets (92, 94) in a machine direction;
advancing a plurality of elastic strands (96) in the machine direction;
stretching the elastic strands (96) in the machine direction;
intermittently adhering the elastic strands (96) between the two layers of continuous sheets (92, 94) to define elastic regions (240) intermittently spaced along the machine direction;
intermittently adhering the two layers of continuous sheets (92, 94) with each other without adhering the elastic strands (96) to define non-elastic regions (230) intermittently spaced along the machine direction and separated by the elastic regions (240) from each other;
advancing the combined two sheets of continuous sheets (92, 94) and elastic strands (96) in the machine direction to a cutting roll system (200) comprising:
an anvil roll (220); and
a cutting roll (210) adjacent the anvil roll, the cutting roll adapted to rotate about a roll axis perpendicular to the machine direction, and wherein the cutting roll (210) comprises a knife block (212); wherein the knife block (212) comprises at least two rows of knife edges (214), each row having at least two knife edges (214) arrayed linearly parallel to the roll axis; each row of knife edges (214) circumferentially spaced apart from each other; each knife edge (214) oriented at an angle which does not match with the roll axis direction (A1) or a roll circumferential direction (C1), the roll circumferential direction (C1) being perpendicular to the roll axis direction (A1);
the knife edges (214) in the same knife block (214) staggered with each other such that each elastic strand (96) in each non-elastic region (230) is cut no more than once;
wherein the cutting roll system (200) comprises at least two knife blocks (212), wherein the knife blocks (212) are further staggered in the machine direction such that no more than one knife block (212) is contacting the anvil roll (220) at the same time.

5. The process of Claim 4 wherein the knife edge (214) is a rotary die cutter.

6. The process of Claim 5 wherein the continuous sheet facing the knife edge (214) is cut through for cutting the elastic strands (96).

7. The process of Claim 4 wherein the elastic strands (96) of the non-elastic region (230) are cut towards the machine direction edge of the non-elastic region (230).

8. The process of Claim 4 wherein a portion of the elastic region (240) further has the two layers of continuous sheets (92, 94) adhered with each other.

9. The process of Claim 4 for simultaneously manufacturing two elastic members (250) adjacent each other in the cross machine direction wherein the two elastic members (250) are made by the same continuous sheets (92, 94), comprising the steps of:
advancing the combined two sheets of continuous sheets (92, 94) and elastic strands 896) to the cutting roll system (200) comprising two knife blocks (212) spaced apart in the cross machine direction, each knife block (212) configured to provide non-elastic regions (230) spaced apart in the cross machine direction; and cutting apart the obtained product along a line in the machine direction.

10. The process of Claim 9 wherein the two elastic members (250) are different in one or more of: length of the continuous sheet (92, 94) in the cross-machine direction, number of elastic strands (96), the interval of elastic strands, stress strain on the elastic strands, type of elastic strands, length of the non-elastic region (230) in the machine direction, length of the non-elastic region (230) in the cross-machine direction, length of the elastic region (240) in the machine direction, and length of the elastic region (240) in the cross-machine direction.

11. The process of Claim 4 for manufacturing an elastic member (250) of an absorbent article (20).

12. The process of Claim 11 wherein the elastic member (250) forms at least a portion of a belt (40) extending transversely about a waist opening (36).

13. The process of Claim 12 wherein the elastic member (250) is a ring-like elastic belt (40), the process further comprising cutting apart the continuous elastic member (250) along a line in the cross-machine direction to form side edges (89F, 89B), and joining the side edges.

14. The process of Claims 9 or 10 wherein the two elastic members (250) are a front belt portion (84) and a back belt portion (86), respectively, of a ring-like elastic belt (40) of an absorbent article (20), the process further comprising cutting the continuous elastic members (250) along a line in the cross-machine direction to form side edges (89F, 89B), and joining the side edges of each other.

15. An absorbent article (20) having a waist opening (36) and two leg openings (34) and continuous in a longitudinal direction and a transverse direction, the article comprising an absorbent main body (38) and a ring-like elastic belt (40), wherein:
the absorbent main body (38) comprises a liquid pervious topsheet (58), a liquid impervious backsheet (60), and an absorbent core (62) disposed therebetween, the absorbent main body (38) has left and right longitudinally continuous side edges (48), front and back transversely continuous end edges (50), longitudinally opposing front and back waist panels (52, 54), and a crotch panel (56) between the waist panels;
the ring-like elastic belt (40) comprises a front belt portion (84) and a back belt portion (86) each comprising an inner sheet (94), an outer sheet (92), and a plurality of elastic strands (96) sandwiched between the inner and outer sheets (94, 92), the elastic strands extending in the transverse direction to provide a continuous elastic ring when the front belt portion (84) and the back belt portion (86) are joined, each front belt portion and back belt portion (84, 86) having transversely continuous proximal and distal edges (88, 90), the proximal edge (90) being located closer than the distal edge (88) relative to the crotch panel (56) of the absorbent main body (38), longitudinally continuous left and right side edges (89F, 89B), a central panel (80F, 80B), and left and right side panels (82F, 82B) contiguous with its central panel, each side panel having a longitudinal length defined by the respective side edge of the respective belt portion;
the central panel (80F) of the front belt portion (84) is joined to the front waist panel (52) of the absorbent main body (38), the central panel (80B) of the back belt portion (86) is joined to the back waist panel (54) of the absorbent main body (38), and the respective left and right side panels (82F, 82B) of the front belt portion and the back belt portion (84, 86) are joined together at or adjacent to the respective left and right side edges (89F, 89B) to form the waist opening (36) and the two leg openings (34);
wherein each of the front belt portion (84) and the back belt portion (86) comprises a non-elastic region (230) where the ring-like elastic belt (40) more or less overlaps with the absorbent core (62), an elastic region (240) defined elsewhere, the non-elastic region (230) having the inner sheet (94) and the outer sheet (92) intermittently adhered with each other without adhering the elastic strands (96), the elastic region (240) having the elastic strands (96) intermittently adhered between the inner sheet (94) and the outer sheet (92), the elastic strands (96) in the non-elastic region (230) being deactivated by cutting the elastic strands (96).

16. The article (20) of Claim 15 wherein the non-elastic region (230) of the ring-like elastic belt (40) is free of any other sheet (92).

17. The article (20) of Claim 15 wherein each of the proximal edges (90) and the distal edges (88) of the front belt portion (84) and the back belt portion (86) are substantially parallel, the longitudinal length of the back belt portion (86) being longer than that of the front belt portion (84), wherein the distal edge (88F) of the front belt portion (84) is aligned with the distal edge (88B) of the back belt portion (86), and the proximal edge (90F) of the front belt portion (84) is not aligned with the proximal edge (90B) of the back belt portion (86).

18. The article (20) of Claim 15 wherein the ring-like elastic belt (40) is manufactured by the process of Claim 4.

19. The article (20) of Claims 15-17 wherein the ring-like elastic belt (40) is manufactured by the process of any of Claims 4-14 using the cutting roll system (200) of any of Claims 1-3.

## Patentansprüche

1. Schneidwalzensystem (200) zum Schneiden einer Vielzahl von elastischen Strängen (96, 216) innerhalb eines Bereichs, wobei das Schneidwalzensystem (200) Folgendes umfasst:
eine Ambosswalze (220); und
eine Schneidwalze (210), angrenzend an die Ambosswalze (220), wobei die Schneidwalze (210) so ausgelegt ist, dass sie um eine Walzenachse dreht, und wobei die Schneidwalze (210) einen Messerblock (212) umfasst;
wobei der Messerblock (212) mindestens zwei Reihen von Messerschneiden (214) umfasst, wobei jede Reihe mindestens zwei Messerschneiden, die linear parallel zur Walzenachse angeordnet sind, aufweist; alle Reihen von Messerschneiden (214) jeweils in Umfangsrichtung voneinander beabstandet sind;
jede Messerschneide (214) in einem Winkel ausgerichtet ist, der nicht mit der Achsenrichtung der Walze (A1) oder einer Walzenumfangsrichtung (C1) übereinstimmt, wobei die Walzenumfangsrichtung (C1) senkrecht zur Richtung der Walzenachse (A1) steht;
die Messerschneiden (214) in demselben Messerblock (212) zueinander versetzt sind, so dass jeder elastische Strang (96, 216) in dem Bereich nur einmal geschnitten wird,
wobei das Schneidwalzensystem (200) eine Vielzahl von Messerblöcken (212) an der Schneidwalze (210) umfasst, wobei die Messerblöcke (212) sowohl in Richtung der Walzenachse (A1) als auch der Walzenumfangsrichtung (C1) zueinander versetzt ausgerichtet sind, so dass immer nur ein Messerblock (212) die Ambosswalze (220) gleichzeitig berührt.

2. Schneidwalzensystem (200) nach Anspruch 1, wobei die Messerschneide (214) eine Rotationsstanze ist.

3. Schneidwalzensystem (200) nach Anspruch 1, wobei die Messerschneiden (214) in demselben Messerblock (212) in demselben Winkel zur Walzenachse ausgerichtet sind.

4. Verfahren zur fortlaufenden Herstellung eines elastischen Elements (250) mit einem elastischen Bereich (240) und einem nicht-elastischen Bereich (230), umfassend die folgenden Schritte:
Vorwärtsbewegen der zwei Schichten aus Endloslagen (92, 94) in einer Maschinenlaufrichtung;
Vorwärtsbewegen einer Vielzahl von elastischen Strängen (96) in der Maschinenlaufrichtung;
Dehnen der elastischen Stränge (96) in der Maschinenlaufrichtung;
diskontinuierliches Verkleben der elastischen Stränge (96) zwischen die zwei Schichten aus Endloslagen (92, 94) zum Definieren von elastischen Bereichen (240), die in Maschinenlaufrichtung diskontinuierlich voneinander beabstandet sind;
diskontinuierliches Verkleben der zwei Schichten aus Endloslagen (92, 94) miteinander, ohne die elastischen Stränge (96) zu verkleben, um nicht-elastische Bereiche (230) zu definieren, die entlang der Maschinenlaufrichtung diskontinuierlich voneinander beabstandet und durch die elastischen Bereiche (240) voneinander getrennt sind;
Vorwärtsbewegen der kombinierten zwei Schichten aus Endloslagen (92, 94) und elastischen Stränge (96) in Maschinenlaufrichtung zu einem Schneidwalzensystem (200), umfassend:
eine Ambosswalze (220); und
eine Schneidwalze (210), angrenzend an die Ambosswalze, wobei die Schneidwalze so ausgelegt ist, dass sie senkrecht zur Maschinenlaufrichtung um eine Walzenachse dreht, und wobei die Schneidwalze (210) einen Messerblock (212) umfasst; wobei der Messerblock (212) mindestens zwei Reihen von Messerschneiden (214) umfasst, wobei jede Reihe mindestens zwei Messerschneiden (214), die linear parallel zur Walzenachse angeordnet sind, aufweist; alle Reihen von Messerschneiden (214) jeweils in Umfangsrichtung voneinander beabstandet sind; jede Messerschneide (214) in einem Winkel ausgerichtet ist, der nicht mit der Achsenrichtung der Walze (A1) oder einer Walzenumfangsrichtung (C1) übereinstimmt, wobei die Walzenumfangsrichtung (C1) senkrecht zur Richtung der Walzenachse (A1) steht;
die Messerschneiden (214) in demselben Messerblock (214) zueinander versetzt sind, so dass jeder elastische Strang (96) in jedem nicht-elastischen Bereich (230) nur einmal geschnitten wird;
wobei das Schneidwalzensystem (200) mindestens zwei Messerblöcke (212) umfasst, wobei die Messerblöcke (212) ferner in Maschinenlaufrichtung versetzt angeordnet sind, so dass immer nur ein Messerblock (212) die Ambosswalze (220) gleichzeitig berührt.

5. Verfahren nach Anspruch 4, wobei die Messerschneide (214) als Rotationsstanze ausgebildet ist.

6. Verfahren nach Anspruch 5, wobei die Endloslage, die zur Messerschneide (214) zeigt, zum Schneiden der elastischen Stränge (96) durchgeschnitten wird.

7. Verfahren nach Anspruch 4, wobei die elastischen Stränge (96) des nicht-elastischen Bereichs (230) in Richtung der Kante in Maschinenlaufrichtung des nicht-elastischen Bereichs (230) geschnitten werden.

8. Verfahren nach Anspruch 4, wobei ein Teil des elastischen Bereichs (240) ferner die zwei miteinander verklebten Schichten aus Endloslagen (92, 94) aufweist.

9. Verfahren nach Anspruch 4 zur gleichzeitigen Herstellung von zwei aneinander angrenzenden elastischen Elementen (250) in Maschinenquerrichtung, wobei die zwei elastischen Elemente (250) aus denselben Endloslagen (92, 94) hergestellt sind, die folgenden Schritte umfassend:
Vorwärtsbewegen der kombinierten zwei Lagen aus Endloslagen (92, 94) und elastischen Strängen (96) zum Schneidwalzensystem (200), das zwei Messerblöcke (212) umfasst, die in Maschinenquerrichtung voneinander beabstandet sind, wobei jeder Messerblock (212) so ausgelegt ist, dass er nicht-elastische Bereiche (230) bereitstellt, die in Maschinenquerrichtung voneinander beabstandet sind; und Zerschneiden des somit erhaltenen Produkts entlang einer Linie in Maschinenlaufrichtung.

10. Verfahren nach Anspruch 9, wobei sich die zwei elastischen Elemente (250) in einem oder mehreren der nachstehenden Aspekte unterscheiden: Länge der Endloslage (92, 94) in Maschinenquerrichtung, Anzahl der elastischen Stränge (96), Intervall der elastischen Stränge, Spannungsverformung der elastischen Stränge, Art der elastischen Stränge, Länge des nicht-elastischen Bereichs (230) in Maschinenlaufrichtung, Länge des nicht-elastischen Bereichs (230) in Maschinenquerrichtung, Länge des elastischen Bereichs (240) in Maschinenlaufrichtung und Länge des elastischen Bereichs (240) in Maschinenquerrichtung.

11. Verfahren nach Anspruch 4 zur Herstellung eines elastischen Elements (250) eines Absorptionsartikels (20).

12. Verfahren nach Anspruch 11, wobei das elastische Element (250) mindestens einen Teil eines Bunds (40) bildet, der quer über eine Taillenöffnung (36) verläuft.

13. Verfahren nach Anspruch 12, wobei das elastische Element (250) als ringförmiger, elastischer Bund (40) ausgeführt ist, wobei das Verfahren ferner das Zerschneiden des elastischen Endloselements (250) entlang einer Linie in Maschinenquerrichtung, um Seitenränder (89F, 89B) zu bilden, und das Verbinden der Seitenränder umfasst.

14. Verfahren nach Anspruch 9 oder 10, wobei die beiden elastischen Elemente (250) als ein vorderer Bundabschnitt (84) und ein hinterer Bundabschnitt (86) eines ringförmigen, elastischen Bunds (40) eines Absorptionsartikels (20) ausgeführt sind, wobei das Verfahren ferner das Schneiden der elastischen Endloselemente (250) entlang einer Linie in Maschinenquerrichtung, um Seitenränder (89F, 89B) zu bilden, und das Verbinden der jeweiligen Seitenränder umfasst.

15. Absorptionsartikel (20), der eine Taillenöffnung (36) und zwei Beinöffnungen (34) aufweist und durchgehend in eine Längsrichtung und eine Querrichtung verläuft, wobei der Artikel einen absorbierenden Hauptkörper (38) und einen ringförmigen, elastischen Bund (40) umfasst, wobei:
der absorbierende Hauptkörper (38) eine flüssigkeitsdurchlässige Oberschicht (58), eine flüssigkeitsundurchlässige Unterschicht (60) und einen dazwischen angeordneten Absorptionskern (62) umfasst, der absorbierende Hauptkörper (38) einen linken und einen rechten längs verlaufenden Seitenrand (48), einen vorderen und einen hinteren quer verlaufenden Endrand (50), in Längsrichtung gegenüberliegende vordere und hintere Taillenfelder (52, 54) und ein Schrittfeld (56) zwischen den Taillenfeldern aufweist;
der ringförmige, elastische Bund (40) einen vorderen Bundabschnitt (84) und einen hinteren Bundabschnitt (86) umfasst, die jeweils eine Innenlage (94), eine Außenlage (92) und eine Vielzahl von elastischen Strängen (96) umfassen, die zwischen der Innen- und der Außenlage (94, 92) angeordnet ist, wobei die elastischen Stränge in Querrichtung verlaufen, um einen durchgehenden elastischen Ring bereitzustellen, wenn der vordere Bundabschnitt (84) und der hintere Bundabschnitt (86) miteinander verbunden werden, wobei jeder vordere Bundabschnitt und hintere Bundabschnitt (84, 86) über querverlaufende, durchgehende proximale und distale Ränder (88, 90) verfügen, wobei der proximale Rand (90) näher als der distale Rand (88) in Bezug auf das Schrittfeld (56) des absorbierenden Hauptkörpers (38) angeordnet ist, sowie in Längsrichtung verlaufende, durchgehende linke und rechte Seitenränder (89F, 89B), ein Mittelfeld (80F, 80B) sowie linke und rechte Seitenfelder (82F, 82B), die mit dem Mittelfeld zusammenhängen, wobei die Längsausdehnung eines jeden Seitenfeldes vom entsprechenden Seitenrand des entsprechenden Bundabschnitts definiert wird;
das Mittelfeld (80F) des vorderen Bundabschnitts (84) mit dem vorderen Taillenfeld (52) des absorbierenden Hauptkörpers (38) verbunden ist, das Mittelfeld (80B) des hinteren Bundabschnitts (86) mit dem hinteren Taillenfeld (54) des absorbierenden Hauptkörpers (38) verbunden ist und die jeweiligen linken und rechten Seitenfelder (82F, 82B) des vorderen Bundabschnitts und des hinteren Bundabschnitts (84, 86) miteinander verbunden sind oder an die jeweiligen linken und rechten Seitenränder (89F, 89B) angrenzen, um die Taillenöffnung (36) und die zwei Beinöffnungen (34) zu bilden;
wobei der vordere Bundabschnitt (84) und der hintere Bundabschnitt (86) jeweils einen nicht-elastischen Bereich (230) umfassen, in dem der ringförmige, elastische Bund (40) den Absorptionskern (62) mehr oder weniger überlappt, wobei an anderer Stelle ein elastischer Bereich (240) definiert ist, im nicht-elastischen Bereich (230) die Innenlage (94) und die Außenlage (92) diskontinuierlich miteinander verklebt sind, ohne mit den elastischen Strängen (96) verklebt zu sein, wobei im elastischen Bereich (240) die elastischen Stränge (96) diskontinuierlich zwischen der Innenlage (94) und der Außenlage (92) verklebt sind, und die elastischen Stränge (96) im nicht-elastischen Bereich (230) durch Zerschneiden der elastischen Stränge deaktiviert sind (96).

16. Artikel (20) nach Anspruch 15, wobei der nicht-elastische Bereich (230) des ringförmigen, elastischen Bunds (40) keine andere Lage aufweist (92).

17. Artikel (20) nach Anspruch 15, wobei der proximale Rand (90) und der distale Rand (88) des vorderen Bundabschnitts (84) sowie des hinteren Bundabschnitts (86) jeweils im Wesentlichen parallel sind, die Längsausdehnung des hinteren Bundabschnitts (86) länger ist als die des vorderen Bundabschnitts (84), wobei der distale Rand (88F) des vorderen Bundabschnitts (84) mit dem distalen Rand (88B) des hinteren Bundabschnitts (86) fluchtet, und der proximale Rand (90F) des vorderen Bundabschnitts (84) nicht mit dem proximalen Rand (90B) des hinteren Bundabschnitts (86) fluchtet.

18. Artikel (20) nach Anspruch 15, wobei der ringförmige, elastische Bund (40) durch das Verfahren nach Anspruch 4 hergestellt ist.

19. Artikel (20) nach den Ansprüchen 15 bis 17, wobei der ringförmige, elastische Bund (40) durch das Verfahren nach einem der Ansprüche 4 bis 14 unter Verwendung des Schneidwalzensystems (200) nach einem der Ansprüche 1 bis 3 hergestellt ist.

## Revendications

1. Système de rouleau de coupe (200) pour couper une pluralité de fils élastiques (96, 216) dans une région, le système de rouleau de coupe (200) comprenant :
un contre-rouleau (220) ; et
un rouleau de coupe (210) adjacent au contre-rouleau (220), le rouleau de coupe (210) conçu pour tourner autour d'un axe de rouleau, et dans lequel le rouleau de coupe (210) comprend un bloc de lames (212) ;
dans lequel le bloc de lames (212) comprend au moins deux rangées de bords de lame (214), chaque rangée ayant au moins deux bords de lame disposés linéairement parallèles à l'axe de rouleau ; chaque rangée de bords de lame (214) étant espacée de façon circonférentielle l'une de l'autre ;
chaque bord de lame (214) étant orienté selon un angle qui ne correspond pas à la direction d'axe de rouleau (A1) ou à une direction circonférentielle de rouleau (C1), la direction circonférentielle de rouleau (C1) étant perpendiculaire à la direction d'axe de rouleau (A1) ;
les bords de lame (214) dans le même bloc de lames (212) étant en quinconce les uns avec les autres de telle sorte que chaque fil élastique (96, 216) dans la région n'est pas coupé plus d'une fois,
dans lequel le système de rouleau de coupe (200) comprend une pluralité de blocs de lames (212) sur le rouleau de coupe (210), les blocs de lames (212) étant en quinconce les uns par rapport aux autres à la fois dans la direction d'axe de rouleau (A1) et dans la direction circonférentielle de rouleau (C1) de telle sorte que pas plus d'un bloc de lames (212) ne vient en contact avec le contre-rouleau (220) en même temps.

2. Système de rouleau de coupe (200) selon la revendication 1, dans lequel le bord de lame (214) est une matrice de coupe rotative.

3. Système de rouleau de coupe (200) selon la revendication 1, dans lequel les bords de lame (214) dans le même bloc de lames (212) sont orientés selon le même angle par rapport à l'axe de rouleau.

4. Procédé de fabrication en continu d'un élément élastique (250) ayant une région élastique (240) et une région non élastique (230) comprenant les étapes consistant à :
faire avancer deux couches de feuilles continues (92, 94) dans une direction de la machine ;
faire avancer une pluralité de fils élastiques (96) dans la direction de la machine ;
étirer les fils élastiques (96) dans la direction de la machine ;
faire adhérer de façon intermittente les fils élastiques (96) entre les deux couches de feuilles continues (92, 94) pour définir des régions élastiques (240) espacées de façon intermittente le long de la direction de la machine ;
faire adhérer de façon intermittente les deux couches de feuilles continues (92, 94) l'une à l'autre sans faire adhérer les fils élastiques (96) pour définir des régions non élastiques (230) espacées de façon intermittente le long de la direction de la machine et séparées par les régions élastiques (240) l'une de l'autre ;
faire avancer les deux feuilles combinées de feuilles continues (92, 94) et de fils élastiques (96) dans la direction de la machine vers un système de rouleau de coupe (200) comprenant :
un contre-rouleau (220) ; et
un rouleau de coupe (210) adjacent au contre-rouleau, le rouleau de coupe conçu pour tourner autour d'un axe de rouleau perpendiculaire à la direction de la machine, et dans lequel le rouleau de coupe (210) comprend un bloc de lames (212) ; dans lequel le bloc de lames (212) comprend au moins deux rangées de bords de lame (214), chaque rangée ayant au moins deux bords de lame (214) disposés linéairement parallèles à l'axe de rouleau ; chaque rangée de bords de lame (214) étant espacée de façon circonférentielle l'une de l'autre ; chaque bord de lame (214) étant orienté selon un angle qui ne correspond pas à la direction d'axe de rouleau (A1) ou à une direction circonférentielle de rouleau (C1), la direction circonférentielle de rouleau (C1) étant perpendiculaire à la direction d'axe de rouleau (A1) ;
les bords de lame (214) dans le même bloc de lames (214) étant en quinconce les uns par rapport aux autres de telle sorte que chaque fil élastique (96) dans chaque région non élastique (230) n'est pas coupé plus d'une fois ;
dans lequel le système de rouleau de coupe (200) comprend au moins deux blocs de lames (212), dans lequel les blocs de lames (212) sont en outre en quinconce dans la direction de la machine de telle sorte que pas plus d'un bloc de lames (212) ne vient en contact avec le contre-rouleau (220) en même temps.

5. Procédé selon la revendication 4, dans lequel le bord de lame (214) est une matrice de coupe rotative.

6. Procédé selon la revendication 5, dans lequel la feuille continue faisant face au bord de lame (214) est tranchée pour couper les fils élastiques (96).

7. Procédé selon la revendication 4, dans lequel les fils élastiques (96) de la région non élastique (230) sont coupés en direction du bord dans la direction machine de la région non élastique (230).

8. Procédé selon la revendication 4, dans lequel une partie de la région élastique (240) a en outre les deux couches de feuilles continues (92, 94) fixées par adhérence l'une avec l'autre.

9. Procédé selon la revendication 4, pour fabriquer simultanément deux éléments élastiques (250) adjacents l'un à l'autre dans la direction transversale de la machine, dans lequel les deux éléments élastiques (250) sont fabriqués par les mêmes feuilles continues (92, 94), comprenant les étapes consistant à :
faire avancer les deux feuilles combinées de feuilles continues (92, 94) et de fils élastiques (96) vers le système de rouleau de coupe (200) comprenant deux blocs de lames (212) espacés dans la direction transversale de la machine, chaque bloc de lames (212) étant configuré pour fournir des régions non élastiques (230) espacées dans la direction transversale de la machine ; et découper le produit obtenu le long d'une ligne dans la direction machine.

10. Procédé selon la revendication 9, dans lequel les deux éléments élastiques (250) sont différents dans un ou plusieurs parmi : la longueur de la feuille continue (92, 94) dans la direction transversale de la machine, le nombre de fils élastiques (96), l'intervalle des fils élastiques, la déformation de contrainte sur les fils élastiques, le type de fils élastiques, la longueur de la région non élastique (230) dans la direction de la machine, la longueur de la région non élastique (230) dans la direction transversale de la machine, la longueur de la région élastique (240) dans la direction de la machine, et la longueur de la région élastique (240) dans la direction transversale de la machine.

11. Procédé selon la revendication 4 pour la fabrication d'un élément élastique (250) d'un article absorbant (20).

12. Procédé selon la revendication 11, dans lequel l'élément élastique (250) forme au moins une partie d'une ceinture (40) s'étendant transversalement autour d'une ouverture de ceinture (36).

13. Procédé selon la revendication 12, dans lequel l'élément élastique (250) est une ceinture élastique de type anneau (40), le procédé comprenant en outre la découpe de l'élément élastique continu (250) le long d'une ligne dans la direction transversale de la machine de façon à former des bords latéraux (89F, 89B), et le raccordement des bords latéraux.

14. Procédé selon la revendication 9 ou 10, dans lequel les deux éléments élastiques (250) sont une partie de ceinture avant (84) et une partie de ceinture arrière (86), respectivement, d'une ceinture élastique de type anneau (40) d'un article absorbant (20), le procédé comprenant en outre la découpe des éléments élastiques continus (250) le long d'une ligne dans la direction transversale de la machine de façon à former des bords latéraux (89F, 89B), et le raccordement des bords latéraux des uns et des autres.

15. Article absorbant (20) ayant une ouverture de ceinture (36) et deux ouvertures de jambe (34) et étant continu dans une direction longitudinale et une direction transversale, l'article comprenant un corps principal absorbant (38) et une ceinture élastique de type anneau (40), dans lequel :
le corps principal absorbant (38) comprend une feuille de dessus perméable aux liquides (58), une feuille de fond imperméable aux liquides (60), et une âme absorbante (62) disposée entre elles, le corps principal absorbant (38) a des bords latéraux longitudinalement continus gauche et droit (48), des bords extrêmes transversalement continus avant et arrière (50), des pans de taille avant et arrière longitudinalement opposés (52, 54), et un pan d'entrejambe (56) entre les pans de taille ;
la ceinture élastique de type anneau (40) comprend une partie de ceinture avant (84) et une partie de ceinture arrière (86) comprenant chacune une feuille interne (94), une feuille externe (92), et une pluralité de fils élastiques (96) intercalés entre les feuilles interne et externe (94, 92), les fils élastiques s'étendant dans la direction transversale pour fournir un anneau élastique continu lorsque la partie de ceinture avant (84) et la partie de ceinture arrière (86) sont jointes, chaque partie de ceinture avant et chaque partie de ceinture arrière (84, 86) ayant des bords proximal et distal transversalement continus (88, 90), le bord proximal (90) étant situé plus près que le bord distal (88) par rapport au pan d'entrejambe (56) du corps principal absorbant (38), des bords latéraux gauche et droit longitudinalement continus (89F, 89B), un pan central (80F, 80B), et des pans latéraux gauche et droit (82F, 82B) contigus par rapport à son pan central, chaque pan latéral ayant une longueur longitudinale définie par le bord latéral respectif de la partie de ceinture respective ;
le pan central (80F) de la partie de ceinture avant (84) est joint au pan de taille avant (52) du corps principal absorbant (38), le pan central (80B) de la partie de ceinture arrière (86) est joint au pan de taille arrière (54) du corps principal absorbant (38), et les pans latéraux gauche et droit respectifs (82F, 82B) de la partie de ceinture avant et de la partie de ceinture arrière (84, 86) sont joints l'un à l'autre au niveau ou à côté des bords latéraux gauche et droit respectifs (89F, 89B) de façon à former l'ouverture de ceinture (36) et les deux ouvertures de jambe (34) ;
dans lequel chacune parmi la partie de ceinture avant (84) et la partie de ceinture arrière (86) comprend une région non élastique (230) où la ceinture élastique de type anneau (40) chevauche plus ou moins l'âme absorbante (62), une région élastique (240) définie ailleurs, la région non élastique (230) ayant la feuille interne (94) et la feuille externe (92) fixées par adhérence de façon intermittente l'une avec l'autre sans faire adhérer les fils élastiques (96), la région élastique (240) ayant les fils élastiques (96) fixés par adhérence de façon intermittente entre la feuille interne (94) et la feuille externe (92), les fils élastiques (96) dans la région non élastique (230) étant désactivés en coupant les fils élastiques (96).

16. Article (20) selon la revendication 15, dans lequel la région non élastique (230) de la ceinture élastique de type anneau (40) est exempte de n'importe quelle autre feuille (92).

17. Article (20) selon la revendication 15, dans lequel chacun des bords proximaux (90) et les bords distaux (88) de la partie de ceinture avant (84) et de la partie de ceinture arrière (86) sont essentiellement parallèles, la longueur longitudinale de la partie de ceinture arrière (86) étant plus longue que celle de la partie de ceinture avant (84), dans lequel le bord distal (88F) de la partie de ceinture avant (84) est aligné sur le bord distal (88B) de la partie de ceinture arrière (86), et le bord proximal (90F) de la partie de ceinture avant (84) n'est pas aligné sur le bord proximal (90B) de la partie de ceinture arrière (86).

18. Article (20) selon la revendication 15, dans lequel la ceinture élastique de type anneau (40) est fabriquée par le procédé selon la revendication 4.

19. Article (20) selon les revendications 15 à 17, dans lequel la ceinture élastique de type anneau (40) est fabriquée par le procédé selon l'une quelconque des revendications 4 à 14, en utilisant le système de rouleau de coupe (200) selon l'une quelconque des revendications 1 à 3.
